Europäisches Patentamt

European Patent Office

Office européen des brevets

(19)

(11) Numéro de publication : **0 189 722**
**B1**

(12) **FASCICULE DE BREVET EUROPÉEN**

(45) Date de publication du fascicule du brevet :
**17.08.88**

(21) Numéro de dépôt : **85402655.6**

(22) Date de dépôt : **31.12.85**

(51) Int. Cl.⁴ : **A 61 K 31/70, A 61 K 47/00**

(54) Composition laxative à base de lactulose, et son procédé de préparation.

(30) Priorité : **15.01.85 FR 8500528**

(43) Date de publication de la demande :
**06.08.86 Bulletin 86/32**

(45) Mention de la délivrance du brevet :
**17.08.88 Bulletin 88/33**

(84) Etats contractants désignés :
**AT BE CH DE FR GB IT LI LU NL SE**

(56) Documents cités :
**FR-A- 2 139 789**
**US-A- 3 860 708**
**US-A- 3 867 524**

(73) Titulaire : **JOUVEINAL S.A.**
**Tour Maine Montparnasse 33 Avenue du Maine**
**F-75755 Paris Cedex 15 (FR)**

(72) Inventeur : **Doat, Bernard Jean-Marie**
**Résidence La Pléiade 67, Boulevard Jacques Millot**
**F-49000 Angers (FR)**
Inventeur : **Letavernier, Jean-François J.M.**
**44, rue Florent Cornillaud**
**F-49000 Angers (FR)**
Inventeur : **Aubard, Gilbert Gustave**
**7, Chemin de la Savetière**
**F-91120 Palaiseau (FR)**
Inventeur : **Llull, Jean Bernard**
**20, rue Saint-Hubert**
**F-91390 Morsang-sur-Orge (FR)**
Inventeur : **Calvet, Alain Pierre**
**10, rue Madame**
**F-78000 Versailles (FR)**
Inventeur : **Junien, Jean-Louis**
**85, Boulevard Suchet**
**F-75016 Paris (FR)**

(74) Mandataire : **Bourgognon, Jean-Marie et al**
**Cabinet Flechner 22, Avenue de Friedland**
**F-75008 Paris (FR)**

**Description**

La présente invention a pour objet une nouvelle composition à base de lactulose, essentiellement destinée au traitement de la constipation chez l'homme.

Le phénomène de la constipation est très courant et affecte un nombre considérable d'individus sans distinction absolue de sexe ni d'âge. Ses causes et ses conséquences en sont d'importances variables. Dans nombre de cas simples, une correction de régime alimentaire suffit à rétablir un état normal.

Cependant, les drogues laxatives sont nécessaires et/ou indispensables dans des cas plus graves comme les constipations fonctionnelles ou lors de situations particulières comme certains examens radiologiques, les suites d'opérations ou d'accouchements de même que pour le traitement des jeunes enfants et des personnes âgées.

Les substances laxatives sont nombreuses et pour certaines d'entre elles connues depuis fort longtemps. Elles peuvent être d'origine végétale, minérale ou de synthèse :

Leur utilisation a pour conséquences des effets désagréables bien connus qu'il est utile d'éliminer.

A cet effet, l'utilisation relativement récente comme substances laxatives de saccharides hydrosolubles non assimilables, comme le lactulose, a amené un progrès indiscutable.

Préparé en 1930, le lactulose ou 4-0-ß-D galactopyranosyl D-glucose, a été proposé vers 1960, sous forme de soluté, pour le traitement de la constipation. De nombreuses études toxicologiques, pharmacologiques, et cliniques ont garanti son innocuité et son efficacité. Le produit sous forme de sirop a été commercialisé dans divers pays (Duphalac en France 1972).

Le lactulose est fabriqué industriellement par isomérisation du lactose sous l'influence de divers agents alcalins. Quel que soit cet agent, le lactulose obtenu est accompagné de sucres apparentés dont les plus importantes sont le galactose et le lactose.

Le produit obtenu se présente sous forme de solution concentrée directement utilisable, avec ou sans adjuvants, pour la préparation de sirops. Ces sirops présentent des inconvénients :

— Leur hyperosmolarité accompagnée d'un goût très sucré et douceâtre est parfois la cause de répulsion et peut conduire à des nausées.

— Dans les cas fréquents de traitements de longue durée, il apparaît fréquemment chez les patients âgés un phénomène de lassitude à l'absorption répétée de ces sirops.

— La posologie des traitements varie d'une part en fonction du sujet auquel il s'adressent (enfants-adultes) et, d'autre part, en fonction de l'individu lui-même. La dose utile est donc particulière au cas et à l'individu et à la mesure d'une quantité même approximative de sirop est sujette à erreur notamment dans le cas de faibles quantités utilisées pour le traitement des jeunes enfants.

Pour remédier à ces inconvénients, il a été tenté de déshydrater les sirops concentrés de lactulose en une poudre.

L'élimination de l'eau par des traitements thermiques brutaux en présence d'adjuvant a été décrite au brevet allemand 27.17.707. On obtient un produit caramélisé dans lequel on peut douter du respect de la quantité et de la nature du lactulose introduit avant traitement.

Des méthodes plus douces ont été décrites, ce sont essentiellement l'atomisation et la lyophilisation.

Au brevet français N°. 2 139 789, on décrit la préparation, par atomisation ou lyophilisation, d'une poudre à haute teneur en lactulose. Afin d'obtenir un produit de manipulation convenable, on fait usage d'un adjuvant : la poudre de farine de Konnyaku, qui représente une charge inactive.

L'utilisation de cet adjuvant nécessite des manipulations préalables qui sont : le broyage de l'adjuvant, dissolution dans l'eau, filtration de la solution.

La solution d'adjuvant ainsi obtenue est ajoutée à un sirop concentré de lactulose de façon à obtenir un mélange de viscosité appropriée pour être atomisé ou lyophilisé.

La poudre obtenue se réhumidifie à l'air ambiant pour réabsorber après 1 mois de 4,5 à 8 % environ d'eau.

Ce procédé nécessite des manipulations peu économiques et ne conduit qu'à un produit sensible à l'humidité et de ce fait inutilisable après un stockage de durée normale.

La lyophilisation sans adjuvant d'un sirop de lactulose ne donne pas un résultat satisfaisant (brevet japonais 52-21063). L'élimination de l'eau n'est pas complète et le produit obtenu par cette technique est hygroscopique, tend à se réhydrater en présence de l'humidité ambiante pour redonner un produit gommeux difficilement manipulable.

Au brevet français N°. 2 392 031, on décrit une amélioration de ce procédé. Elle consiste, à partir d'un produit obtenu par lyophilisation à éliminer l'eau résiduelle par un traitement à l'éthanol absolu non dénaturé, ce qui permet d'obtenir une poudre de lactulose manipulable et non hygroscopique.

Ce traitement a, en raison du prix de l'éthanol absolu, une incidence sur le prix déjà élevé du produit obtenu par lyophilisation et d'autre part nécessite un séchage parfait de la pourdre pour éliminer les traces d'éthanol résiduel. Si ce stade est effectué d'une manière insuffisante, le malade traité peut absorber des quantités non négligeables d'éthanol.

Aux brevets des Etats-Unis d'Amérique numéros 3 860 708 et 3 867 524, on propose d'administrer à l'homme du sirop à 50 % en poids de lactulose pour éliminer le sulfate de baryum retenu dans l'intestin après absorption de ce produit comme opacifiant, lors de certains examens radiologiques, et pour traiter

2

les affectations consécutives aux périodontites. Dans ces deux brevets, on mentionne, de manière identique, sous la forme d'une clause de style, que le lactulose peut être présenté, non seulement sous la forme préférée d'un sirop, mais aussi sous d'autres formes galéniques parmi lesquelles figurent des bonbons gélifiés. On propose à cet effet d'utiliser de la pectine comme agent gélifiant, de l'acide sorbique et ses sels comme agents de conservation, et les acides citrique et tartrique comme agents organoleptiques. Sans autres précisions, le galéniste en est amené à conclure que ces bonbons gélifiés auront été préparés, s'ils l'ont été, par action d'une pectine d'usage classique dans l'industrie pharmaceutique sur du sirop de lactulose. Dans cette pectine, utilisée habituellement en pharmacie et dite « fortement estérifiée », plus de 50 % des groupes carboxyliques de l'acide polygalacturonique sont estérifiés par le méthanol.

La demanderesse a effectué des essais de gélification de ce type. Le mélange de sirop de lactulose normal, c'est-à-dire ayant une teneur en matières sèches hydrosolubles inférieure à 63 % en poids, et de pectine classique ne permet pas d'obtenir une gelée. La gélification n'a lieu que si la teneur en matières sèches hydrosolubles du lactulose est portée à une valeur supérieure à 65 % en poids. Or, comme le confirme indirectement la revendication 2 du brevet 3.867.524, La teneur maximale en matières sèches hydrosolubles d'un sirop normal de lactulose est de 63 %. Il s'ensuit que, ou bien en fait on n'a pas préparé de bonbons gélifiés, ce qui est le plus probable, ou bien on a fait subir au préalable au sirop de lactulose une évaporation pour augmenter sa teneur en matières sèches.

Evaporer du sirop de lactulose nécessite de le porter à une température supérieure à 100° C et cette évaporation s'accompagne nécessairement d'une dénaturation du lactulose.

Quels que soient les procédés décrits ou supposés de préparation d'une composition à base de lactulose. Ceux-ci font appel à des technologies élaborées qui nécessitent un équipement coûteux.

L'invention rompt avec cet état de la technique jalonné par les brevets mentionnés ci-dessus qui tendaient à obtenir du lactulose en poudre ou sous forme pseudo-solide.

L'invention a pour objet un procédé de préparation d'une composition laxative à base de lactulose, caractérisé en ce qu'il consiste :

a) à porter du sirop aqueux de lactulose, ayant une teneur en matières sèches hydrosolubles inférieure à 63 % en poids, à une température comprise entre 20 et 90° C,

b) à ajouter au sirop de lactulose un sel hydrosoluble de calcium ou de magnésium à raison de 0,1 à 1 % en poids par rapport au poids du sirop et un agent d'ajustement du pH pharmaceutique acceptable en une quantité telle que le pH du sirop additionné du sel et de l'agent d'ajustement soit compris entre 2,5 et 5, le stade b pouvant être effectué avant, pendant ou après le stade a, puis

c) à ajouter au sirop, porté entre 20 et 90° C et additionné du sel et de l'agent d'ajustement du pH, de 0,1 à 5 % en poids par rapport au poids du sirop d'une pectine ayant un pourcentage des groupes carboxyliques estérifiés inférieur à 50, puis

d) à agiter l'ensemble du sirop et de la pectine pendant au moins cinq minutes, puis

e) à laisser l'ensemble du sirop et de la pectine refroidir jusqu'à une température où se produit une prise de gelée.

On obtient ainsi une composition à base de lactulose qui se présente sous la forme d'une gelée ayant une viscosité à 20 °C au viscosimètre Brookfields comprise entre 15 000 et 30 000 centipoises et un indice de réfraction à 20 °C compris entre 1,430 et 1,445 et un pH compris en 3 et 4.

On a renoncé ainsi à la présentation de la composition de lactulose sous forme solide. Mais, en renonçant à cet état, au bénéfice d'un état gélifié visqueux semblable à une confiture, qui se tranche facilement, non seulement on obtient, pour l'essentiel, toutes les propriétés requises, notamment de stabilité et de mise sous forme de doses, mais encore on a la possibilité supplémentaire d'une utilisation sous forme masquée. Compromettre sur l'état solide apporte ainsi des avantages sans aucun inconvénient, alors que l'art antérieur s'était acharné maladroitement à viser cet état solide.

Le premier stade du procédé suivant l'invention de préparation d'une composition laxative à base de lactulose consiste à porter du sirop de lactulose ayant une teneur en matières sèches hydrosolubles inférieure à 63 % en poids, et une température comprise entre 20 et 90 °C.

Les sirops concentrés de lactulose ont une teneur en matières sèches hydrosolubles habituellement comprise entre 50 et 63 % en poids de matières sèches hydrosolubles. Ils se présentent sous la forme de sirops limpides, jaune pâle, de densité voisine de 1,310. Le lactulose proprement dit représente, en général, de 45 à 55 % du poids du sirop. Il est accompagné d'autres substances apparentées, telles que lactose et épilactose, galactose, lévulose et tagatose, représentant habituellement de 5 à 12 % du poids du sirop.

On préfère porter le sirop à une température comprise entre 50 et 75° C, de manière à fluidifier suffisamment le sirop sans cependant se rapprocher trop de la température de dénaturation.

Le deuxième stade du procédé suivant l'invention consiste à ajouter au sirop de lactulose un sel hydrosoluble de calcium ou de magnésium, à raison de 0,1 à 1 % en poids par rapport au poids du sirop. On préfère tout particulièrement les sels de calcium et notamment le chlorure de calcium et le lactate de calcium.

On ajoute également au sirop un agent d'ajustement du pH pharmaceutiquement acceptable, en une quantité telle que le pH du sirop additionné du sel et de l'agent d'ajustement soit compris entre 2,5 et 5 et, de préférence, entre 3 et 4. Comme agent d'ajustement du pH, on préfère utiliser des acides organiques et, tout particulièrement, l'acide citrique ou l'acide tartrique, en raison de leurs propriétés organoleptiques. On ajoute par exemple de 0,1 à 1 % en poids d'agent d'ajustement du pH pour 100 parties en poids de sirop.

On peut intervertir le premier stade et le deuxième stade du procédé suivant l'invention ou les effectuer en même temps.

Le troisième stade du procédé suivant l'invention consiste à ajouter au sirop, porté entre 20 et 90 °C et additionné du sel et de l'agent d'ajustement du pH, de 0,1 à 5 % en poids par rapport au poids du sirop d'une pectine ayant un pourcentage des groupes carboxyliques estérifiés inférieur à 50.

Les pectines sont des produits carbohydratés obtenus généralement à partir des extraits acides dilués de pulpe de citron ou de pomme. Elles sont également présentes dans les parois cellulaires de végétaux comme les fruits. Les racines, comme les carottes et les betteraves, et également des tubercules comme les pommes de terre en contiennent également. Les pectines sont définies chimiquement comme des esters méthyliques partiels d'acides polygalacturoniques dont le poids moléculaire peut atteindre 200 000.

Parmi ces pectines, l'invention fait appel non pas à des pectines très estérifiées comme on en utilise habituellement dans l'industrie pharmaceutique, mais à des pectines faiblement estérifiées obtenues notamment par hydrolyse partielle des fonctions ester des pectines fortement estérifiées sous l'action de l'ammoniaque. Elles sont caractérisées par un pourcentage d'estérification des fonctions carboxyliques inférieur à 50 % et, de préférence, compris entre 18 % et 39 %. On détermine le pourcentage d'estérification suivant la méthode définie à U.S.P. XXI-NF XVI 1985, page 790. Les pectines faiblement estérifiées présentent des fonctions amide. Leur indice d'amidification est en général compris entre 12 et 25 %. Cet indice représente le pourcentage de fonction carboxylique transformé en fonction amide lors du traitement par l'ammoniaque. On trouvera un procédé de détermination de cet indice dans FAO Food and nutrition paper (specifications for identity and purity of carrier solvents emulsifiers and stabilizeurs enzymes preparations flavoring agents food colours sweetening agents and other food additives) Genève, 23 mars au 1er avril 1981.

Le quatrième stade du procédé suivant l'inventaire consiste à agiter l'ensemble du sirop et de la pectine pendant au moins 5 minutes et, généralement, pendant 15 minutes à 2 heures, pour obtenir une bonne homogénéité.

Le dernier stade du procédé suivant l'invention consiste à laisser l'ensemble du sirop et de la pectine refroidir jusqu'à une température où se produit la prise en gelée et, notamment, jusqu'à la température ambiante de 15 °C à 25 °C, étant entendu cependant qu'une température inférieure ou même légèrement supérieure peut convenir également.

On obtient ainsi une composition laxative qui, de préférence, a une viscosité comprise entre 18 000 et 25 000 centipoises et un indice de réfraction compris entre 1,435 et 1,440. Cette composition à base de lactulose comprend habituellement de 35 à 45 % en poids de lactulose, de 40 à 55 % en poids d'eau, une quantité suffisante d'un réseau pectinocalcique, pour donner à la composition la texture d'une gelée ayant les viscosités indice de réfraction et pH indiquées précédemment.

De préférence, la composition comprend une quantité d'un arôme suffisante pour masquer le goût du lactulose. Les arômes sont choisis pour identifier le mieux possible les gelées avec des produits naturels, tant du point de vue de l'aspect que du point de vue de l'odeur et de la saveur. On utilise, le plus souvent, à cet effet les arômes de cassis, groseille, framboise, pruneau, coing, orange, citron, mandarine, figue, etc.

L'addition d'un produit antifongique permet de garantir une bonne conservation de la gelée. A cet effet, on préfère utiliser l'acide sorbique et, plus particulièrement, le sorbate de potassium en des quantités représentant de 0,01 à 0,5 % en poids par rapport au poids de la composition.

Les exemples suivants illustrent l'invention.

### Exemple 1

Pour illustrer de façon précise la préparation d'une composition préférée, suivant l'invention, on donne dans ce qui suit la préparation d'une «venue» de 200 kg dont la composition, pour 100 grammes, est la suivante :

| | |
|---|---|
| — sirop de lactulose à 50 % (58 % de matières sèches hydrosolubles) | 80,992 g |
| — acide citrique | 0,350 g |
| — lactate de calcium | 0,108 g |
| — pectine faiblement estérifiée de type A | 0,700 g |
| (ayant un pourcentage d'estérification de 33 %, un pourcentage d'amidation de 16 % et un pH de la solution de 1 % de 4,7) | |
| — arôme naturel type «pruneau» hydrosoluble | 1,000 g |
| — sorbate de potassium | 0,050 g |
| — eau purifiée | 16,800 g. |

On prépare une solution par dispersion de 1,40 kg de pectine dans 33,60 kg d'eau préalablement

**0 189 722**

portée à 70° C. Après 15 à 20 minutes d'agitation, on obtient une solution homogène.

Parallèlement, dans une «turbosphère», on introduit 162,00 kg de sirop de lactulose à 50 %. On chauffe ce sirop sous agitation à 70° C, puis on introduit successivement en dispersant 216,00 g de lactate de calcium, puis 100 g de sorbate de potassium.

Après dissolution de ces produits dans le sirop à 70° C, on introduit petit à petit, en laissant dissoudre entre chaque ajout, 700 g d'acide citrique. Après cette addition, le pH du mélange est de 3,5 environ. On introduit ensuite en 10 minutes environ, la solution de pectine préalablement préparée. On maintient l'agitation pendant 10 minutes, puis on introduit 2,00 kg d'arôme pruneau dans le mélange. On laisse refroidir jusqu'à 25° C. On conditionne la gelée obtenue en doses unitaires de 8 à 50 grammes.

La gelée a une viscosité de 20° C au viscosimètre Brookfiels de 22 000 centipoises, et un indice de réfraction à 20° C de 1,438. Cette gelée a un aspect et une texture qui la fait ressembler à une gelée alimentaire. Elle se tranche facilement et nettement à la cuillère et n'adhère pratiquement pas aux parois des récipients de verre et de matière plastique. Sa rigidité en permet une manipulation facile. La gelée n'est pas coulante, mais toutefois suffisamment souple pour être agréable gustativement au palais. La conservation de la gelée pendant une année et demie à la température ambiante n'altère ni son aspect, ni ses propriétés organoleptiques. Cette gelée conserve son aspect translucide et sa texture jusqu'à une température de 35° C environ. Elle tend à se liquéfier au-dessus de 40° C, mais reprend son état physique initial par refroidissement.

#### Exemple 2

On reprend l'exemple 1, mais la formulation est la suivante :

| | |
|---|---|
| — sirop de lactulose à 50 % (58,0 % de matières sèches hydrosolubles) | 81,40 g |
| — acide citrique | 0,25 g |
| — chlorure de calcium dihydraté | 0,05 g |
| — pectine de type B ayant un pourcentage d'estérification de 31 %, un pourcentage d'amidon de 17 % et un pH de la solution à 1 % de 4,9 | 0,70 g |
| — arôme soluble type pruneau | 0,80 g |
| — sorbate de potassium | 0,50 g |
| — eau | 16,30 g. |

On obtient un produit ayant un aspect et des propriétés physiques semblables à ceux de l'exemple 1.

#### Exemple 3

On reprend l'exemple 2, si ce n'est que la pectine est une pectine de type C ayant un pourcentage d'estérification de 21 %, un pourcentage d'amidation de 23 %, et un pH de la solution aqueuse à 1 % de 4,6.

La composition a un aspect et des propriétés organoleptiques semblables à celles de l'exemple 2, la gelée tend à se liquéfier à partir de 30° C, mais reprend toutefois son aspect initial par refroidissement à 15-20° C.

#### Exemple 4 (comparatif)

On reprend l'exemple 1, mais avec la formulation suivante, pour 100 grammes :

| | |
|---|---|
| — sirop de lactulose à 50 % | 81,00 g |
| — acide citrique | 0,35 g |
| — sorbate de potassium | 0,05 g |
| — gomme xanthane | 1,00 g |
| — arôme de pruneau | 1,00 g |
| — eau | 16,60 g. |

La composition obtenue a une texture pseudo-gélifiée gommeuse. La préhension en est malaisée, le produit colle à la cuillère, sur le verre et sur la matière plastique, d'une façon semblable à du miel.

#### Exemple 5 (comparatif)

On reprend l'exemple 4 en remplaçant la gomme xanthane par 2,00 g de gomme de caroube.

La composition obtenue n'est pas une gelée. Elle a une texture visqueuse et gluante de préhension très difficile. Elle dégage une odeur désagréable.

#### Exemple 6 (comparatif)

5

On reprend l'exemple 5, si ce n'est que l'on remplace la gomme de caroube par 1,00 g de gélatine « 250 Bloom ». On trouvera la définition du degré Bloom qui caractérise la force de gélification de la gélatine dans USP XXI NF XVI,1985, page 1329.

La composition obtenue a une consistance trop dure pour être prélevée facilement à la cuillère. Une coloration brune apparaît et progresse dans le temps. Cette coloration brune à soi seule, due à une réaction de Maillard entre les fonctions aminées protéiques de la gélatine et les fonctions hydroxylées du lactulose rend cette composition inutilisable.

### Exemple 7 (comparatif)

On reprend l'exemple 4, si ce n'est que l'on remplace la gomme de xanthane par 1,20 g d'une pectine ayant un pourcentage d'estérification de 65 %.

La composition de lactulose obtenue n'est pas sous la forme d'une gelée, mais a une consistance très sirupeuse. Elle colle à la cuillère sur les parois de verre et de matière plastique.

### Exemple 8 (comparatif)

On reprend l'exemple 7, en ajoutant 0,80 g de lactate de calcium.

La composition obtenue a un aspect, une consistance et des propriétés semblables à celles de l'exemple 7.

Le conditionnement des gelées suivant l'invention peut être effectué dans des récipients conventionnels d'un type utilisé pour les confitures. Ces gelées à base de lactulose se prêtent particulièrement à la présentation sous forme de doses unitaires, présentation peu pratique pour la forme sirop ou incertaine avec la forme poudre, à cause de l'hydroscopicité potentielle de celle-ci.

La présentation sous forme de doses unitaires présente des avantages indéniables comme :
— assurance de la dose absorbée,
— contamination des prises multiples évitée,
— conservation des doses inutilisées lors d'un arrêt de traitement,
— facilité et sécurité d'emploi dues à la texture de la forme gelée,
— possibilités d'utilisation sous formes masquées : sur tartines, dans yaourts, etc.,
— facilité de division des doses en particulier pour le traitement des jeunes enfants,
— diminution de la monotonie lors des traitements au long cours en variant les parfums des gelées, etc.,
— meilleure conservation du produit, notamment vis-à-vis des phénomènes d'oxydation, qui dénaturent le goût des arômes et la coloration des produits.

Le conditionnement en doses unitaires est aisément réalisé dans des godets de type «unidose» obtenus par thermoformage de matériaux plastiques de qualités appropriées.

La gelée peut être répartie en doses unitaires comprises entre 8 et 50 g. Les doses unitaires de 8 g, qui correspondent à environ 3,5 g de lactulose (40 %) sont particulièrement indiquées au traitement de la constipation chez les jeunes enfants, les doses de 25 g environ à des traitements d'entretien chez l'adulte et les doses de 50 g à des traitements d'attaque ou dans les cas de constipations sévères.

La posologie est de 1,75 à 60 g de lactulose par jour selon l'âge du patient et la sévérité de la constipation à traiter. Ces doses sont aisément mesurables à partir des conditionnements en doses unitaires précédemment décrits.

La composition à base de lactulose objet de la présente invention est un produit nouveau de présentation attrayante, utile pour traiter le phénomène de la constipation dans les cas les plus divers, et plus particulièrement chez les enfants, les sujets âgés, les suites d'accouchement ou d'opération, en particulier après hémorroïdectomies, les interventions pré ou post-radiologiques, dans certains cas de constipations d'origine médicamenteuse.

## Revendications

1. Procédé de préparation d'une composition laxative à base de lactulose, caractérisé en ce qu'il consiste :

a) à porter du sirop aqueux de lactulose, ayant une teneur en matières sèches hydrosolubles inférieure à 63 % en poids, à une température comprise entre 20 et 90° C,

b) à ajouter au sirop de lactulose un sel hydrosoluble de calcium ou de magnésium à raison de 0,1 à 1 % en poids par rapport au poids du sirop et un agent d'ajustemnt du pH pharmaceutiquement acceptable en une quantité telle que le pH du sirop additionné du sel et de l'agent d'ajustement soit compris entre 2,5 et 5, le stade b pouvant être effectué avant, pendant ou après le stade a, puis

c) à ajouter au sirop, porté entre 20 et 90° C et additionné du sel et de l'agent d'ajustement du pH, de 0,1 à 5 % en poids par rapport au poids du sirop d'une pectine ayant un pourcentage des groupes carboxyliques estérifiés inférieur à 50, puis

d) à agiter l'ensemble du sirop et de la pectine pendant au moins cinq minutes, puis

6

e) à laisser l'ensemble du sirop et de la pectine refroidir jusqu'à une température où se produit une prise en gelée.

2. Procédé suivant la revendication 1, caractérisé en ce qu'il consiste à porter le sirop aqueux à une température comprise entre 50 et 75 °C.

3. Procédé suivant la revendication 1 ou 2, caractérisé en ce que le sirop aqueux de lactulose a une teneur en matières sèches hydrosolubles comprise entre 50 et 63 % en poids.

4. Procédé suivant l'une des revendications précédentes, caractérisé en ce que le sel de calcium ou de magnésium est un chlorure ou un lactate.

5. Procédé suivant l'une des revendications précédentes, caractérisé en ce que l'agent d'ajustement du pH est de l'acide citrique ou de l'acide tartrique.

6. Procédé suivant l'une des revendications précédentes, caractérisé en ce que le pH du sirop additionné du sel et de l'agent d'ajustement est compris entre 3 et 4.

7. Procédé suivant l'une des revendications précédentes, caractérisé en ce qu'il consiste à utiliser de la pectine ayant un pourcentage des groupes carboxyliques estérifiés compris entre 18 et 39 %.

8. Procédé suivant l'une des revendications précédentes, caractérisé en ce qu'il consiste à utiliser de la pectine ayant un degré d'amidification compris entre 12 et 25 %.

9. Procédé suivant l'une des revendications précédentes, caractérisé en ce qu'il consiste à agiter l'ensemble du sirop et de la pectine pendant une durée comprise entre 15 minutes et 2 heures.

10. Procédé suivant l'une des revendications précédentes, caractérisé en ce qu'il consiste à laisser l'ensemble du sirop et de la pectine refroidir jusqu'à la température ambiante, comprise entre 15 et 25 °C.

11. Composition laxative à base de lactulose, caractérisée en ce qu'elle est préparée par un procédé suivant l'une des revendications précédentes et se présente sous la forme d'une gelée ayant une viscosité, à 20 °C, au viscosimètre Brookfields, comprise entre 15 000 et 30 000 centipoises, un indice de réfraction, à 20 °C, compris entre 1,430 et 1,445 et un pH compris entre 3 et 4.

12. Composition suivant la revendication 11, caractérisée en ce qu'elle a une viscosité comprise entre 18 000 et 25 000 centipoises et un indice de réfraction compris entre 1,435 et 1,440.

13. Composition laxative à base de lactulose, caractérisée en ce qu'elle comprend : de 35 à 45 % en poids de lactulose, de 40 à 55 % en poids d'eau, une quantité suffisante d'un réseau pectinocalcique pour donner à la composition la texture d'une gelée ayant une viscosité à 20 °C au viscosimètre Brookfields, comprise entre 15 000 et 30 000 centipoises, et un indice de réfraction à 20 °C, compris entre 1,430 et 1,445, et une quantité suffisante d'un agent d'ajustement du pH pour que le pH de la composition soit compris entre 3 et 4.

14. Composition suivant l'une des revendications 11 à 13, caractérisée en ce qu'elle comprend un arôme en une quantité suffisante pour masquer le goût du lactulose.

15. Composition suivant l'une des revendications 11 à 14, caractérisée en ce qu'elle est présentée en doses unitaires de 8 à 50 grammes, contenant chacune de 35 à 45 % en poids de lactulose.

**Claims**

1. A process for the preparation of a lactulose-based laxative composition, characterized in that it comprises :

a) heating an aqueous lactulose syrup with a watersoluble dry substance content below 63 % by weight to a temperature between 20 and 90 °C,

b) adding to the lactulose syrup a watersoluble calcium or magnesium salt in an amount of 0.1 to 1 % by weight compared with the weight of the syrup and a pharmaceutically acceptable pH-adjusting agent in an amount such that the pH of the syrup to which the salt and adjusting agent have been added is between 2.5 and 5, whereby stage b) can be performed before, during of after stage a), then

c) adding to the syrup, heated between 20 and 90 °C and to which the salt and pH-adjusting agent have been added, 0.1 to 5 % by weight with respect to the syrup weight of a pectin having a percentage of esterified carboxylic groups below 50, then,

d) stirring the syrup and pectin for at least 5 minutes and then,

e) leaving the syrup and pectin to cool to a temperature where jellification takes place.

2. A process according to claim 1, characterized in that it comprises heating the aqueous syrup to a temperature between 50 and 75 °C.

3. A process according to claims 1 or 2, characterized in that the aqueous lactulose syrup has a watersoluble dry substance content between 50 and 63 % by weight.

4. A process according to any one of the preceding claims, characterized in that the calcium or magnesium salt is a chloride or lactate.

5. A process according to any one of the preceding claims, characterized in that the pH-adjusting agent is citric or tartaric acid.

6. A process according to any one of the preceding claims, characterized in that the pH of the syrup to which the salt and adjusting agent have been added is between 3 and 4.

7. A process according to any one of the preceding claims, characterized in that it comprises using pectin with a percentage of esterified carboxylic groups between 18 and 39 %.

7

8. A process according to any one of the preceding claims characterized in that it comprises using pectin with a degree of amidification between 12 and 25 %.

9. A process according to any one of the preceding claims, characterized in that it comprises stirring the syrup and the pectin for between 15 minutes and 2 hours.

10. A process according to any one of the preceding claims, characterized in that it comprises allowing the syrup and pectin to cool to ambiant temperature between 15 and 25 °C.

11. A lactulose-based laxative composition, characterized in that it is prepared by a process according to any one of the preceding claims and in the form of a jelly with a viscosity at 20 °C measured on the Brookfield viscosimeter between 15,000 and 30,000 centipoises, a refractive index at 20 °C between 1.430 and 1.445 and a pH between 3 and 4.

12. A composition according to claim 11, characterized in that it has a viscosity between 18,000 and 25,000 centipoises and a refractive index between 1.435 and 1.440.

13. A lactulose-based laxative composition, characterized in that it comprises 35 to 45 % by weight of lactulose, 40 to 55 % by weight of water, an adequate quantity of a pectinocalcium system to give the composition the texture of a jelly with a viscosity measured on the Brookfield viscosimeter at 20 °C between 15,000 and 30,000 centipoises and a refractive index at 20 °C of between 1.430 and 1.445 and an adequate quantity of a pH-adjusting agent to ensure that the pH of the composition is between 3 and 4.

14. A composition according to any one of the claims 11 to 13, characterized in that it comprises a flavouring agent in an adequate quantity to mask the taste of the lactulose.

15. A composition according to any one of the claims 11 to 14, characterized in that it is in the form of 8 to 50 gram unit doses, each containing 35 to 45 % by weight of lactulose.


**Patentansprüche**

1. Verfahren zur Herstellung einer laxativen Zusammensetzung auf Basis von Lactulose, bei dem man :

a) wässrigen Lactulosesirup, der einen Gehalt an wasserlöslichem Trockenmaterial unter 63 Gew.% besitzt, bei einer Temperatur zwischen 20 und 90 °C hält,

b) in den Laktulosesirup ein wasserlösliches Calcium- oder Magnesiumsalz zu 0,1 bis 1 Gew.%, bezogen auf das Gewicht des Sirups und ein pharmazeutisch annehmbares pH-Wert Einstellungsmittel in einer Menge zugibt, daß der pH des Sirups mit dem Salz und dem pH-Einstellungsmittel zwischen 2,5 und 5 beträgt, wobei der Schritt b) vor, während oder nach Schritt a) durchgeführt werden kann, anschließend

c) gibt man zum Sirup, den man zwischen 20 und 90 °C hält und der das Salz und das pH-Einstellungsmittel enthält, 0,1 bis 5 Gew.%, bezogen auf das Gewicht des Sirups, ein Pektrin, das weniger als 50 % veresterte Carboxylgruppen enthält, anschließend

d) rührt man das ganze für mindestens 5 Minuten, dann

e) läßt man das ganze bis zu einer Temperatur, bei der das Produkt geliert, abkühlen.

2. Verfahren nach Anspruch 1, dadurch gekennzeichnet, daß man den wässrigen Sirup bei einer Temperatur zwischen 50 und 75 °C hält.

3. Verfahren nach Anspruch 1 oder 2, dadurch gekennzeichnet, daß der wässrige Lactulosesirup einen wasserlöslichen getrockneten Feststoffgehalt zwischen 50 und 63 Gew.% aufweist.

4. Verfahren nach einem der vorhergehenden Ansprüche, dadurch gekennzeichnet, daß das Calcium- oder Magnesiumsalz ein Chlorid oder ein Lactat ist.

5. Verfahren nach einem der vorhergehenden Ansprüche, dadurch gekennzeichnet, daß das pH-Einstellungsmittel Zitronensäure oder Weinsäure ist.

6. Verfahren nach einem der vorhergehenden Ansprüche, dadurch gekennzeichnet, daß der pH-Wert des Sirups, der das Salz und das pH-Einstellungsmittel enthält, zwischen 3 und 4 liegt.

7. Verfahren nach einem der vorhergehenden Ansprüche, dadurch gekennzeichnet, daß man ein Pektin verwendet, das zwischen 18 und 39 % veresterte Carboxylgruppen aufweist.

8. Verfahren nach einem der vorhergehenden Ansprüche, dadurch gekennzeichnet, daß man ein Pektin verwendet, das einen Amidierungsgrad zwischen 12 und 25 % aufweist.

9. Verfahren nach einem der vorhergehenden Ansprüche, dadurch gekennzeichnet, daß man die Mischung aus Sirup und Pektin für eine Dauer zwischen 15 Minuten und 2 Stunden rührt.

10. Verfahren nach einem der vorhergehenden Ansprüche, dadurch gekennzeichnet, daß man die Mischung aus Sirup und Pektin auf Raumtemperatur zwischen 15 und 25 °C abkühlen läßt.

11. Laxative Zusammensetzung auf Basis von Lactulose, dadurch gekennzeichnet, daß man sie nach einem Verfahren gemäß einem der vorhergehenden Ansprüche herstellt und daß sie in Form eines Gelees mit einer Viskosität bei 20 Grad, gemessen im Brookfield's Viskosimeter, zwischen 15 000 und 30 000 Centipoise, einem Refraktionsindex bei 20 °C zwischen 1,430 und 1,445 und einem pH-Wert zwischen 3 und 4, vorliegt.

12. Zusammensetzung nach Anspruch 11, dadurch gekennzeichnet, daß sie eine Viskosität im Bereich zwischen 18 000 und 25 000 Centipoise und einen Refraktionsindex zwischen 1,435 und 1,440 aufweist.

13. Laxative Zusammensetzung auf Basis von Lactulose, enthaltend : 35 bis 45 Gew.% Lactulose, 40 bis 55 Gew.% Wasser, eine ausreichende Menge eines Calcium-Pektinnetzwerkes um der Zusammensetzung die Struktur eines Gels zu geben, das eine Viskosität bei 20 °C im Brookfields Viskosimeter zwischen 15 000 und 30 000 Centipoise, einen Refraktionsindex bei 20 °C zwischen 1,430 und 1,445 aufweist sowie eine ausreichende Menge eines pH-Regulierungsmittels, so daß der pH-Wert der Zusammensetzung zwischen 3 und 4 liegt.

14. Zusammensetzung nach einem der Ansprüche 11 bis 13, dadurch gekennzeichnet, daß sie einen Geschmacksstoff in einer ausreichenden Menge enthält um den Geschmack der Lactulose zu überdecken.

15. Zusammensetzung nach einem der Ansprüche 11 bis 14, dadurch gekennzeichnet, daß sie in Form von Einzeldosen mit 8 bis 50 g, jeweils enthaltend 35 bis 40 Gew.% Lactulose, vorliegt.